# EUROPEAN PATENT APPLICATION

(11) **EP 4 000 569 A1**
(43) Date of publication of application: **25.05.2022**
(21) Application number: 20207551.1
(22) Date of filing: 13.11.2020
(51) Int. Cl.: A61F 11/06, G10L 15/00, A41D 13/05, A61F 9/06, F16P 1/00, G10L 15/22

(54) **PERSONAL PROTECTIVE DEVICE WITH LOCAL VOICE RECOGNITION AND METHOD OF PROCESSING A VOICE SIGNAL THEREIN**

(71) Applicant: 3M Innovative Properties Company, Saint Paul, MN 55133-3427 (US)
(72) Inventor: Kurzhals, Mr. Holger, 47839 Krefeld (DE); Stalder, Mr. Michael, 47589 Uedem (DE); Hjort, Mr. Patrick, 33142 Värnamo (SE); Johansson, Mr. Magnus, 554 38 Jonkoping (SE)
(74) Representative: Vollmers, Hans-Gerd

(57) **Abstract**

The present disclosure relates to a personal protective device 100, 100', 300, 400, 500 with local voice recognition comprising a voice receiving unit 20, 20', a voice processing unit 30, 30', a command processing unit 40, 40' and a control unit 42, 42'. The present disclosure further relates to a method of processing a voice signal 202, 202' in a personal protective device 100, 100', 300, 400, 500 with local voice recognition comprising the steps of recognizing a voice signal 202, 202' by a microphone 10, 10', transmitting the voice signal 202, 202' to the voice processing unit 30, 30', optionally of an external device 31', processing the received voice signal 202, 202' with the voice processing unit 30, 30' to generate a voice command based on the received voice signal 202, 202', processing the voice command generated from the voice signal 202, 202' and matching it with the command information stored within the voice processing unit 30, 30' to generate a voice command information block, transmitting the voice command information block to the command processing unit 40, 40', processing the voice command information block and matching it with the control information stored within the command processing unit 40, 40' to generate a control command and transmitting the control command to the control unit 42, 42'.

## Description

The present disclosure relates to a personal protective device with local voice recognition and to a method of processing a voice signal therein.

Personal protective equipment or devices have been widely used to protect users while working in dangerous or hazardous environments. These may include noisy areas, areas with extreme temperatures, areas with atmospheres containing hazardous or poisonous ingredients, areas with intensive light emissions such as welding arcs, areas with injury risk such as construction work zones.

Personal protective devices have been developed and refined over time and may include electrical or electronic functions improving the protection and the comfort, respectively, of the wearers. Such personal protective devices may include hearing protection devices having a noise cancelling function, welding shields with automatic darkening filters, respiratory devices with valves or active ventilation. These functions may require adjustments, which were typically done by the user pushing buttons on such devices, e. g. by hand. This may become difficult or even problematic if the work of the user requires both hands so that operating the buttons may be difficult or impossible. Also, hygiene aspects may be important, e. g. for respirators being used in a contaminated environment.

Voice recognition systems have been developed to control functions of electrical or electronic devices by voice signals or voice commands. One advantage of voice-controlled devices is that users can control functions of these in a so-called hands-free operation, i. e. not needing to push button or the like to initiate switching of functions. Examples of these systems would be online based systems as commercially available for controlling functions of a computer or of home equipment such as lighting or consumer electronics. Such systems are typically artificial intelligence based and/or require, because of some complexity and variety of controls and commands leading to reasonably high amount of data and processing effort, to be connected to external processing devices such as servers providing these functions. Typically, these connections are provided by a Wide Area Network (WAN) or by the internet. Although the success probability of these systems recognizing the words spoken by a user may be sufficiently high, these systems may be disadvantageous in local applications, in particular where no connection to a network, i. e. a WAN or the internet, is available. However, such situations may occur during work and in working environments where users use personal protective devices.

Therefore, a need exists for personal protective devices working without WAN or internet connection, in particular devices performing local voice recognition. It is therefore an object of the present disclosure to improve the safety and comfort of users of personal protective device by providing voice recognition locally.

The present disclosure relates to a personal protective device with local voice recognition comprising a voice receiving unit comprising at least one microphone for recognizing voice signals of a user. The personal protective device comprises a control unit for controlling functions of the personal protective device and a voice processing unit for processing voice signals received from the at least one microphone. The voice processing unit is connected to the at least one microphone of the voice receiving unit, wherein the voice processing unit is configured to generate a voice command based on the received voice signal. The voice processing unit comprises predetermined command information stored internally within the voice processing unit. The voice processing unit is configured to match the voice command generated from the voice signal with the stored command information to generate a voice command information block. The personal protective device comprises a command processing unit for processing the voice command information block generated by the voice processing unit. The command processing unit is connected to the voice processing unit and to the control unit. The command processing unit comprises predetermined control information stored within the command processing unit. The command processing unit is configured to match the voice command information block with the stored control information to generate a control command and to transmit the control command to the control unit. Such a personal protective device is advantageous because a reliable voice recognition can even be performed when no WAN or internet connection is available to the personal protective device. In other words, the personal protective device operates in an autarkic mode. Also, the data security is increased as no data from the user is being sent to remote networks. Furthermore, a personal protective device with a local voice recognition allows operating the personal protective device in a hands-free mode, which minimizes or avoids doffing of the personal protective device while operating it and while switching functions thereon. This may further enhance safety and comfort for the user of the personal protective device.

The microphone of the voice receiving unit may be a digital microphone or an analogue microphone. In the latter case the further signal processing is digital, an AD/DA converter may be required. If the further signal processing is analogue, no converter may be needed, unless the microphone is digital, then an DA/AD converter may be needed.

Connections between components or units of the personal protective device, e. g. between the microphone, the voice receiving unit, the voice processing unit, the command processing unit, the control unit and/or the feedback unit including the indication units, may, for example, be electrically or optically. The connection may be wired, e. g. electrical wires, optical fibers or wirelessly, e. g. of the type Radio Frequency Identification (RFID), Bluetooth, Bluetooth Low Energy (BLE), ZigBee, Near Field Communication (NFC), Near Field Magnetic Induction (NFMI) or other suitable wireless connections. Also, light signals wirelessly sent are conceivable for these connections.

The present disclosure further relates to a method of processing a voice signal in a personal protective device with local voice recognition. The personal protective device comprises a voice receiving unit comprising at least one microphone for recognizing voice signals of a user. The personal protective device comprises a control unit for controlling functions of the personal protective device and a voice processing unit for processing voice signals received from the at least one microphone of the voice receiving unit. The voice processing unit is connected to the at least one microphone of the voice receiving unit, wherein the voice processing unit is configured to generate a voice command based on the received voice signal. The voice processing unit comprises predetermined command information stored internally within the voice processing unit. The voice processing unit is configured to match the voice command generated from the voice signal with the stored command information to generate a voice command information block. The personal protective device comprises a command processing unit for processing the voice command information block generated by the voice processing unit, wherein the command processing unit is connected to the voice processing unit and to the control unit, wherein the command processing unit comprises predetermined control information stored within the command processing unit and wherein the command processing unit is configured to match the voice command information block with the stored control information to generate a control command and to transmit the control command to the control unit. The method comprises the steps of recognizing a voice signal by a microphone, transmitting the voice signal to the voice processing unit, optionally of an external device, processing the received voice signal with the voice processing unit to generate a voice command based on the received voice signal, processing the voice command generated from the voice signal and matching it with the command information stored within the voice processing unit to generate a voice command information block, transmitting the voice command information block to the command processing unit, processing the voice command information block and matching it with the control information stored within the command processing unit to generate a control command and transmitting the control command to the control unit. Such a method of processing a voice signal in a personal protective device is advantageous because a reliable voice recognition can even be performed when no WAN or internet connection is available to the personal protective device. In other words, the personal protective device operates in an autarkic mode. Also, the data security is increased as no data from the user is being sent to remote networks. The method provides for operating the personal protective device in a hands-free mode including switching functions thereon. As mentioned above, avoiding doffing the personal protective device may enhance safety and comfort of the device and its operation.

Personal protective devices are designed to protect the wearer or user of it from hazards due to injury or infection. The hazards to a wearer or user include physical, light, electrical, heat, chemical, biohazard or airborne particulate matter hazards. Personal protective devices typically include protective clothing, helmets, goggles or other garments or equipment designed to protect a wearer or user such as visors, shields, helmets, masks or respirators.

A Wide Area Network or Connection (WAN) is a telecommunication network extending over a large geographic area for the purpose of computer networking. This includes exchange and/or processing of data between different devices. Typically, internet connections are WAN connections. In contrast thereto is a LAN (local area network), which is described below.

A Local Area Network (LAN) is a computer network to connect computers or other electronic devices within a limited area. This limited area may be building, part of a building, a property or part of a property. In contrast to a WAN, the geographical extension is much smaller. The typical maximum distance within a LAN may be 100 m. Also, in contrast to a WAN, a LAN connects a lower number of computers and/or electronic devices with each other. A LAN may be formed wired or wirelessly. The latter is also known as WLAN (wireless local area network) or as WIFI. Typically, WLAN or WIFI networks work based on the IEEE 802.11 standard. In a LAN, typically the latency time is lower compared to a WAN. A LAN or WLAN may be available and accessible by the personal protective device even if a WAN or the internet is not available or accessible.

Offline mode is an operational mode where a device is not connected to a WAN or to the internet. The required processing and/or storage capabilities necessary for operating the device are built into the device or may be present in an external device connected to or connectable to the personal protective device via a short-range connection. Such an external device may preferably be a smartphone.

Local voice recognition in the sense of the present disclosure is understood as a system for voice recognition being run in an offline mode. In other words, the device performing such a local voice recognition is not connected to a WAN or to the internet and may either have all required components or units built into the device or may have voice and/or command processing components or units outsourced to an external device not connected to a WAN or to the internet, but connected via a short-range connection including a LAN or WLAN connection to the personal protective device. Online mode or online voice recognition is understood as a voice recognition system where the processing of the voice signals is done at remote processing capabilities, which may be connected to the device via a WAN or internet connection.

Short-range transmissions or connections within the sense of the present disclosure are wired or wireless transmissions or connections between devices over typically 1 - 2 m, 2 - 5 m or 5 - 10 m distance. A short-range transmission may also include a LAN or WLAN transmission.

An external device, unit or component in the sense of the present disclosure is understood as a separate device or component, i. e. in a physical distance from another device, which is connected via a wired or wireless connection. Such an external device may include smartphones or external control or processing devices. The external device as such may be connected to a WAN or to the internet, but not for processing operations within the voice recognition.

An internal device, unit or component in the sense of the present disclosure includes separate hardware components within the device for different functionalities or a common hardware component running software for different functionalities.

A voice signal in the sense of the present disclosure is a signal spoken by a user of a personal protective device. The signal comprises acoustic waves forming a single number, character or a number of characters defining a word or a series of a few words defining a keyword.

A voice command in the sense of the present disclosure is a voice signal which was matched with an entry of a list of possible voice signals. In other words, a voice command is generated based on a voice signal, which means that the voice signal is converted into a voice command. Such a list may be stored in a personal protective device. In other words, the voice command represents a voice signal which was recognized by a personal protective device being a specific word, phrase or sentence. A voice command information block in the sense of the present disclosure is understood as a list of suggestions of possible commands based on a voice signal as received by a microphone and a voice receiving unit and matched with an entry of a list of possible voice signals. The list of suggestions may comprise more than one option for a specific action to be initiated at an electronic device. In other words, it contains a list of possibilities for the device. The list does not only comprise the possibilities, but also a probability of what was recognized where a confidence level value CLV was assigned, e. g. based on a voice signal "ABC", the list may comprise "ABC" CLV = 9000, "ABE" CLV = 8000, "EBC" CLV = 5000 and so on. It may be important to assign a threshold CLV under which possibilities may be ignored by the system. For example, to switch a device comprising a radio from one station to another, the user may speak "channel 1" or "station 1" leading to the same results, i. e. that the device with its radio switches to "channel 1".

A control command in the sense of the present disclosure is a command which was matched with entry of a list of possible controls for a personal protective device, i. e. the control information, and which is transmitted to a control unit for initiation of certain action of an electronic device. The control command may be obtained by matching the entries of the voice command information block with predetermined control information representing the possibilities for a specific command possible for a specific device. This includes the possibility that more than one entry of that list leads to the same control command. In order to realize this, the command processing unit and the control unit of a personal protective device with local voice recognition needs to be harmonized and synchronized with regard to the control commands possible for that specific device.

A control unit within the sense of the present disclosure is understood as a unit to initiate action within a personal protective device. For example, changing the operation mode of the device, changing setting on the device etc. will be initiated by the control unit. Typically, the control unit was operated by switch buttons or the like to be pushed by the user.

It is understood by the skilled person that the above-mentioned parts or components such as the microphone, the voice receiving unit, the voice and/or command processing unit, the control unit and/or the feedback unit with the audible, visual and/or haptic indication unit may be separate parts within the personal protective device. It is also conceivable, that some parts or components are integral with each other, for example it is conceivable that the voice receiving unit and the voice and/or command processing unit are built on one microchip. It is also conceivable that one microchip runs software to represent the function of a voice receiving unit, a voice and/or command processing unit, a control unit and/or a feedback unit. In the latter case, the dividing into the different components is rather seen from a functional aspect and not necessarily alone from a constructional or structural aspect.

Providing feedback to the user of the personal protective device is understood as an indication provided to the user about the match of the voice command with the stored command information and about the match of the voice command information block with stored command information of the command processing unit. The feedback may include a positive feedback or indication about the match, a negative feedback or indication that there was no match or a combination of both. It is also conceivable that the feedback or indication includes a repetition of the voice signal as spoken by the user. Also, the feedback may include an indication to the user about the control command to be transmitted to the control unit and may include an indication about the action to be initiated by the control unit or that no action is initiated. The feedback may also include an indication, e. g. an audible indication, which is different from a voice signal as spoken by the user, but which is clearly assignable by the user. It is understood that the terms feedback and indication are likewise used for an action where the user is made aware of a certain state or change of the personal protective device.

A personal protective device according to the present disclosure can be made by providing a voice receiving unit and a voice and/or command processing unit as described above and below, e. g. arranged on a printed circuit board comprising circuitry for voice receiving and voice and/or command processing, and arrange voice receiving unit and the voice and/or command processing unit at the personal protective device. One example may include arranging the voice receiving unit and the voice and/or command processing unit within or at the housing of the personal protective device. Typically, the connection from or to the voice and/or command processing unit may be wired, but it is also conceivable to arrange that connection wirelessly. Another example may include arranging the voice receiving unit at the personal protective device, whereas the voice and/or command processing unit may be arranged in a short-range, e. g. typically 0,3 or 1 - 2 m, 2 - 5 m or 5 - 10 m distance spaced, from the personal protective device. In the latter case, the voice and/or command processing unit may be connected to the personal protective device wired or wirelessly.

In one embodiment, the personal protective device further comprises a feedback unit comprising at least one indication means to provide a feedback to a user of the personal protective device. The feedback unit is connected to the control unit. The control unit is configured to control the at least one feedback unit such that an indication is provided to the user about the matching of the voice command with the stored command information and/or about the matching of the voice command information block with the stored control information and/or about the control command to be transmitted to the control unit. Such a feedback unit is advantageous because the user of the personal protective device is informed about a match by the feedback unit. This may help the user with using the personal protective device, in particular as doffing of the personal protective device is minimized or avoided because the user is informed about the switching operation and does not need to look manually e. g. at buttons or displays or lamps of the device. This may be of particular importance for workers in hazardous environments.

In another certain embodiment, the feedback unit of the personal protective device comprises an audible indication unit to provide an audible indication to a user. Such an audible indication unit is advantageous because an audible indication may help the user still focusing on certain visual aspects while receiving the feedback or indication. In other words, a deflection from what the user needs to see in the surrounding may be minimized or avoided thereby.

In yet a further certain embodiment, the audible indication unit of the personal protective device comprises at least one speaker electrically connected to the control unit for playing an acoustic signal to a user as audible indication. The control unit is configured to control the feedback unit such that the feedback unit generates an acoustic signal to be played on the at least one speaker. Such an audible indication unit is advantageous because a speaker playing an acoustic signal to the user represents an easy and reliable way of providing an audible feedback or indication to the user.

In a further certain embodiment, the feedback unit of the personal protective device comprises a visual indication unit to a provide visual indication to a user. Such a visual indication unit is advantageous because a visual feedback or indication may help to inform the user about the match even in noisy environments where audible feedback may be missed.

In yet another certain embodiment, the feedback unit of the personal protective device comprises a haptic indication unit to provide a haptic indication to a user. Such a haptic indication unit is advantageous because a haptic feedback may help to avoid input conflicts with visual and/or audible input given to the user of the personal protective device. In other words, if the user needs to listen to audio signals and needs to read visual signals, a haptic feedback or indication is not in conflict with these other signals.

In one embodiment, the voice processing unit and/or command processing unit of the personal protective device is contained within the personal protective device. Such a processing unit is advantageous because no extra device is necessary to perform the voice recognition with the personal protective device. A robust and easy design of a system for the voice recognition is thereby provided if all necessary parts or components are contained within the personal protective device.

In another embodiment, the voice processing unit and/or command processing unit of the personal protective device is arranged remote from the personal protective device. The personal protective device is configured to communicate with the voice and/or command processing unit via a short-range connection. Such a connection may, for example, include wired or wireless transmission or connections between devices over typically 1 - 2 m, 2 - 5 m or 5 - 10 m distance and be electrically or optically. Such a connection may be wired, e. g. electrical wires, optical fibers or wirelessly, e. g. of the type Radio Frequency Identification (RFID), Bluetooth, Bluetooth Low Energy (BLE), ZigBee, Near Field Communication (NFC), Near Field Magnetic Induction (NFMI) or other suitable wireless connections. Such a voice and/or processing unit is advantageous because an external device close to the personal protective device may provide for an improved processing capability compared to a built-in processing capability within the personal protective device. This may lead to an improved voice recognition with regard to accuracy, speed or the like.

In a further embodiment, the voice processing unit of the personal protective device comprises storage space for storing additional command information different from the predetermined command information and/or wherein the command processing unit of the personal protective device comprises storage space for storing additional control information. For example, such additional storage space may be used to store new voice commands and/or new control commands. Such a processing unit is advantageous because the additional command information may allow for an extended range of command and/or functions.

In yet a further embodiment, the personal protective device is a hearing protection device with local voice recognition further comprising two earmuffs or ear plugs and noise reduction means, optionally a headband. Alternatively, the two earmuffs or the ear plugs may be attached to a hard hat or protective helmet without a headband. Such a personal protective device is advantageous because the user can operate the personal protective device without pushing operation buttons or knobs. This would allow the user to operate the personal protective device in a so-called hands-free mode, which may help to avoid disruptions of what the user of such a personal protective device is doing, e. g. working in a noisy environment. Also, doffing of the personal protective device in a noisy environment may be minimized or avoided thereby.

In another embodiment, the personal protective device is a welding shield with local voice recognition further comprising an automatic darkening filter. Such a personal protective device is advantageous because the user can operate the personal protective device without pushing operation buttons or knobs. This would allow the user to operate the personal protective device in a so-called hands-free mode, which may help to avoid disruptions of what the user of such a personal protective device is doing, e. g. welding work. Also, doffing of the personal protective device in an environment requiring protection against welding arcs may be minimized or avoided thereby.

In yet another embodiment, the personal protective device is a respiratory device with local voice recognition further comprising filter cartridges for filtering inhaled air. Such a personal protective device is advantageous because the user can operate the personal protective device without pushing operation buttons or knobs. This would allow the user to operate the personal protective device in a so-called hands-free mode, which may help to avoid disruptions or what the user of such a personal protective device is doing, e. g. working in an environment where respiratory protection is required because of the conditions of the ambient atmosphere. Also, doffing of the personal protective device in such an environment may be minimized or avoided thereby.

In still another embodiment, the voice processing unit of the personal protective device, preferably the control unit, is further configured to process a keyword training mode for training a user about the correct pronunciation of the voice signals. For such a training mode, components of the personal protective device may be used, e. g. a loudspeaker, to play a voice command or keyword to the user. It is also conceivable that additional components are arranged to facilitate such a training mode, in particular in case the personal protective device does not yet comprise the components required for the training mode. Such a processing unit is advantageous because by training the user in using the system the accuracy may be improved, i. e. the confidence level of the recognized voice signal or command may be increased. Also, the system may use limited command information only which may help to overcome limited processing and/or storage capabilities of the personal protective device compared to systems with an online voice recognition.

In a further embodiment, the method of processing a voice signal in a personal protective device further comprises the step of providing an indication to the user about the matching of the voice command with the stored command information and/or about the matching of the voice command information block with the stored control information and/or about the control command to be transmitted to the control unit, wherein the indication to the user comprises an audible indication, a visual indication, a haptic indication or a combination thereof. Such a method is advantageous because the user of the personal protective device is informed about a match by the feedback unit. This may help the user with using the personal protective device.

The invention was described in various embodiments above. It is understood by a person skilled in the art, that one of, several of or all the above-mentioned embodiments can be combined with each other.

The invention will now be described in more detail with reference to the following Figures exemplifying particular embodiments of the invention:

### Brief description of the Figures

Fig. 1 is a schematic illustration of the method of processing a voice signal in a personal protective device with local voice recognition according to the present disclosure.
Fig. 2A is a schematic illustration of a personal protective device with local voice recognition according to the present disclosure.
Fig. 2B is a schematic illustration of a personal protective device with local voice recognition according to another embodiment of the present disclosure.
Fig. 3 is a perspective view of a personal protective device with local voice recognition according to an embodiment of the present disclosure.
Fig. 4 is a perspective view of a personal protective device with local voice recognition according to another embodiment of the present disclosure.
Fig. 5 is a perspective view of a personal protective device with local voice recognition according to a further embodiment of the present disclosure.

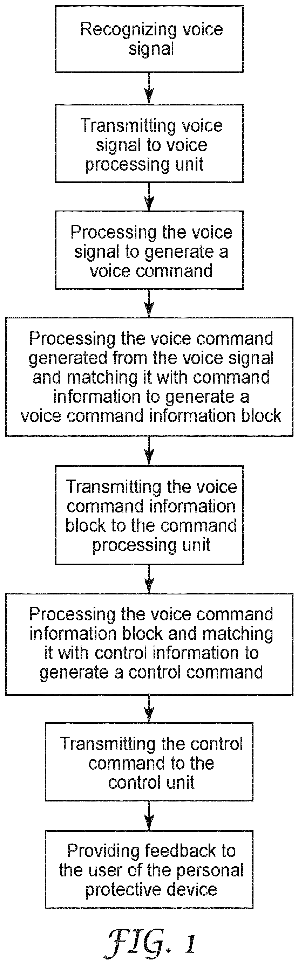

Fig. 1 illustrates in a schematic block diagram the method of processing a voice signal in a personal protective device with local voice recognition according to the present disclosure. A user (not shown here) speaks a voice signal, which is recognized by the voice receiving unit 20, 20' (not shown here). The received voice signal is transmitted to the voice processing unit 30, 30' (also not shown here). The transmitted voice signal is then processed within the voice processing unit 30 30' to generate a voice command. The voice command is then matched with the command information stored within the voice processing unit 30, 30' to generate a voice command information block. The voice command information block is then transmitted to and processed within the command processing unit 40, 40' (not shown here). This includes matching the voice command information block with the command information stored within the command processing unit 40, 40' to generate a control command. The control command is then transmitted to the control unit 42, 42' and feedback is provided to the user of the personal protective device about the match.

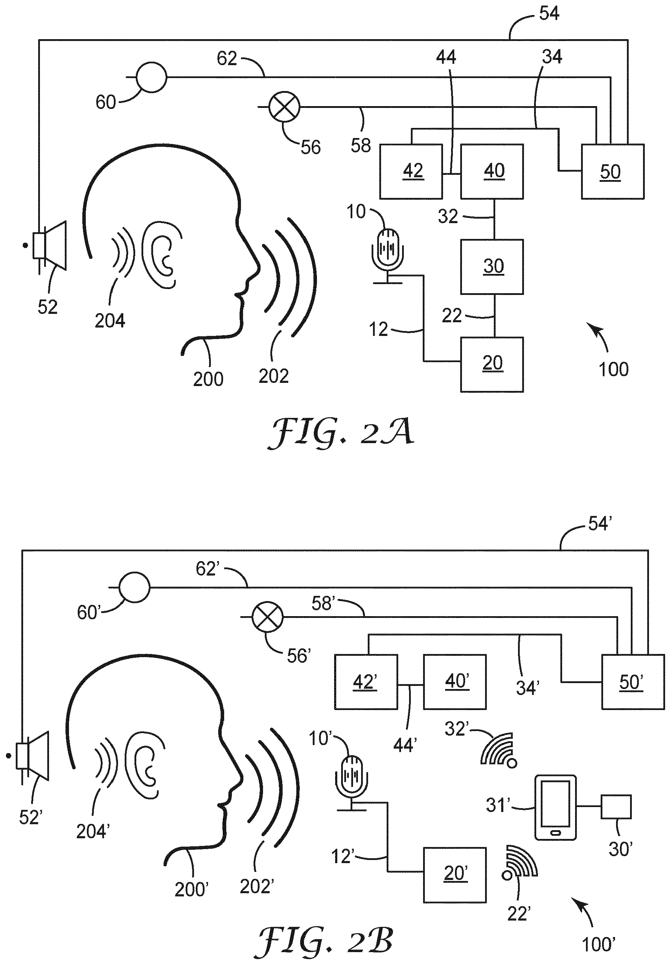

Fig. 2A shows one embodiment of the personal protective device 100 with local voice recognition and a user 200 using the personal protective device 100. The user 200 speaks a voice signal 202 which is received by the microphone 10 of the voice receiving unit 20 which is connected via connection 12. The voice receiving unit 20 may convert the received voice signal into a digital voice signal, if the microphone 10 is an analogue microphone. In case of using a digital microphone, this step may be omitted. The voice receiving unit 20 transmits the voice signal 202 via connection 22 to the voice processing unit 30. There, the voice signal 202 is processed including generating a voice command based on the received voice signal 202 and matching the voice command with the predetermined command information stored within the voice processing unit 30 to generate a voice command information block. The voice command information block is then transmitted to the command processing unit 40 via connection 32. In the command processing unit 40, the voice command is being processed including matching the voice command with the stored control information and generating a control command. The control command is then transmitted to the control unit 42 via connection 44 for initiating an action in the personal protective device 100, e. g. turning up or down of the speaker volume, switching on or off additional functions such as noise cancellation, radio, one or two way communication to other personal protective devices (not shown here) or the like. In parallel, the control unit 42 transmits a feedback signal about the match to the feedback unit 50 via connection 34. The feedback unit 50 may initiate an audible indication to the user 200 by an audible indication unit 52, e. g. a loudspeaker 52, connected via connection 54. Also, the feedback unit 50 may initiate a visual indication to the user 200 by a visual indication unit 56, e. g. a lamp or LED (light emitting diode) 56 or the like, connection via connection 58. The feedback unit 50 may also initiate a haptic indication to the user 200 by a haptic indication unit 60, e. g. a vibration alert generated by a motor 60 or the like, connected via connection 62.

Fig. 2B shows another embodiment of the personal protective device 100' with local voice recognition and a user 200' using the personal protective device 100'. The personal protective device 100' is similar to the personal protective device 100 as shown in Fig. 1 except that the voice processing unit 30' is part of an external device 31' such as smartphone 31' or the like. In the embodiment shown, the external device 31' is wirelessly connected to the voice receiving unit 20' via wireless connection 22'. Also, a wired connection (not shown) is conceivable. The external device 31' is also wirelessly connected to the command processing unit 40' via wireless connection 32'. Also, a wired connection (not shown) is conceivable. The command processing unit 40' is connected to the feedback unit 50' via connection 34' on the other hand. These wireless connections may be of the type Radio Frequency Identification (RFID), Bluetooth, Bluetooth Low Energy (BLE), ZigBee, Near Field Communication (NFC), Near Field Magnetic Induction (NFMI) or other suitable wireless connections. In the embodiment shown in Fig. 2B, the user 200' speaks a voice signal 202' which is received by the microphone 10' of the voice receiving unit 20' which is connected via connection 12'. The voice receiving unit 20' may convert the received voice signal into a digital voice signal, if the microphone 10' is an analogue microphone. In case of using a digital microphone, this step may be omitted. The voice receiving unit 20' transmits the voice signal 202' via wireless connection 22' to the voice processing unit 30'. There, the voice signal 202' is processed including generating a voice command based on the received voice signal 202' and matching the voice command with the predetermined command information stored within the voice processing unit 30' to generate a voice command information block. The voice command information block is then transmitted to the command processing unit 40' via wireless connection 32'. In the command processing unit 40', the voice command information block is being processed including matching the voice command information block with the stored control information and generating a control command. The control command is then transmitted to the control unit 42' via connection 44' for initiating an action in the personal protective device 100', e. g. turning up or down of the speaker volume, switching on or off additional functions such as noise cancellation, radio, one or two way communication to other personal protective devices (not shown here) or the like. In parallel, the control unit 42' transmits a feedback signal about the match to the feedback unit 50' via connection 34'. The feedback unit 50' may initiate an audible indication to the user 200' by an audible indication unit 52', e. g. a loudspeaker 52' or a piezo element, connected via connection 54'. Also, the feedback unit 50 may initiate a visual indication to the user 200' by a visual indication unit 56', e. g. a lamp or LED (light emitting diode) 56' or the like, conneced via connection 58'. The feedback unit 50' may also initiate a haptic indication to the user 200' by a haptic indication unit 60', e. g. a vibration alert generated by a motor 60' or the like, connected via connection 62'.

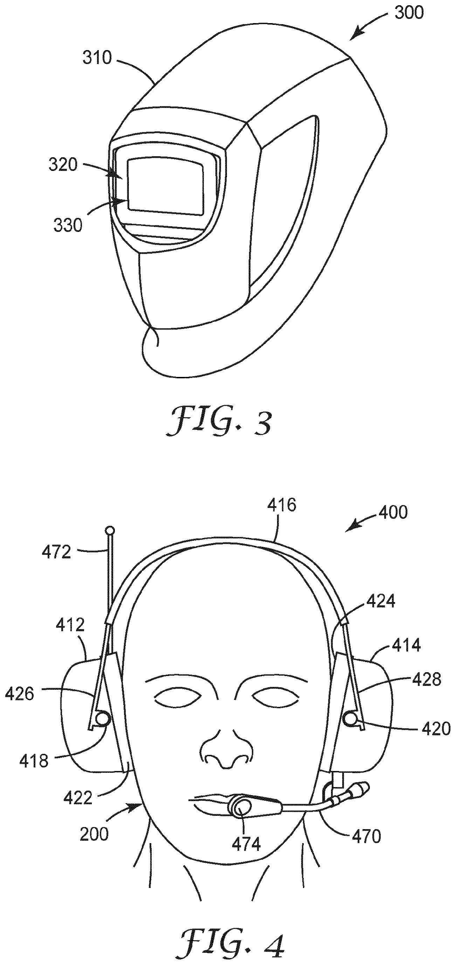

Fig. 3 shows in a front-side perspective view an embodiment of the personal protective device 300 with local voice recognition according to an embodiment of the present disclosure which is a welding helmet 300 with local voice recognition. The welding helmet 300 comprises a welding helmet shell 310 and an automatic darkening filter 330, which is mounted in a forward-facing optically transmissive window 320. The welding helmet 300 comprises the components of the personal protective device 100 or personal protective device 100' as laid out above in Figs. 2A or 2B, which have been omitted in Fig. 3 for simplification (for details see Figs. 2A or 2B). The control unit 42, 42' (not shown here) is connected to the automatic darkening filter 330 or other switchable electronic components such that switching of the automatic darkening filter 330 and/or of these other components can be performed.

Fig. 4 shows in a perspective view a user 200 wearing a personal protective device 400 with local voice recognition according to an embodiment of the present disclosure being a hearing protection device 400 with local voice recognition. The hearing protection device 400 comprises a first and a second cup 412, 414 mounted to a headband 416 via mounts 418, 420. Mounts 418, 420 are connected to headband mount 426, 428 formed by a section of the headband 416. An antenna 472 extends from one of the cups 412 for allowing improvement of a signal transmission, for example of an electrical voice signal from or to the hearing device 400. Fig. 4 further shows a microphone boom 470 extending from one of the cups 414 towards the voice microphone 474 such that voice microphone 474 is held in a position close to the user's mouth. A good sound transfer of the mouth to the voice microphone 474 when the user is speaking is thereby achieved. As can be seen in Fig. 4, ear cushions 422, 424 are arranged between the cups 412, 414 and the user's head such that a good wearing comfort of the hearing protection device is achieved. The hearing protective device 400 comprises the components of the personal protective device 100 or personal protective device 100' as laid out above in Figs. 2A or 2B, which have been omitted in Fig. 4 for simplification (for details see Figs. 2A or 2B). The control unit 42, 42' (not shown here) is connected to components within the hearing protection device 400, e. g. a microphone, a loudspeaker, a noise reduction unit or other switchable electronic components, such that switching of these components can be performed.

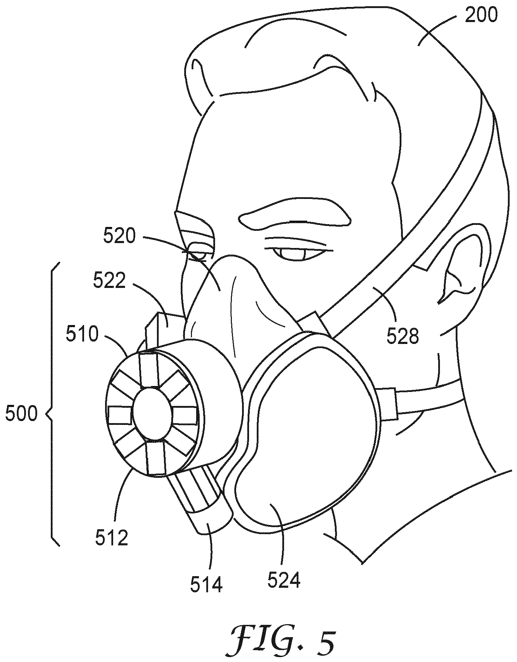

Fig. 5 shows in a perspective view a user 200 wearing a personal protective device 500 with local voice recognition according to an embodiment of the present disclosure being respiratory device 500 with local voice recognition. The respiratory device 500 comprises an axial fan 512 which is contained inside a housing 516. To control the operation of the axial fan 512, a switch mechanism 514 is connected to the control unit 42, 42' (not shown here), e. g. to optimize the desired speed of the axial fan, and hence, the cooling effect based upon the environmental conditions, the task the wearer 200 is undertaking, and the wearer's personal choice. The personal protection respiratory device 500 that is illustrated in Figure 5 is a 3M^{™} 4251 Valved Filtering Half Face Respirator. As shown in Figure 5, a pair of filter cartridges 522, 524 are integrally attached to the respirator mask 520 at respective inhalation ports (not shown). Each of the inhalation ports includes a respective inhalation valve (not shown) on the inside of the respirator mask 520 which open as a wearer 200 draws a breath. The respirator mask 520 has adjustable straps 528 for attachment to the wearer 200. The respiratory device 500 comprises the components of the personal protective device 100 or personal protective device 100' as laid out above in Figs. 2A or 2B, which have been omitted in Fig. 5 for simplification (for details see Figs. 2A or 2B). The control unit 42, 42' (not shown here) is connected to components within the respiratory device 500, e. g. a motorized ventilation unit or other switchable electronic components, such that switching of these components can be performed.

## Claims

1. A personal protective device (100, 100', 300, 400, 500) with local voice recognition comprising
a. a voice receiving unit (20, 20') comprising at least one microphone (10, 10') for recognizing voice signals (202, 202') of a user (200, 200'),
b. a control unit (42, 42') for controlling functions of the personal protective device (100, 100', 300, 400, 500),
c. a voice processing unit (30, 30') for processing voice signals (202, 202') received from the at least one microphone (10, 10'),
- the voice processing unit (30, 30') is connected to the at least one microphone (10, 10') of the voice receiving unit (20, 20'),
- wherein the voice processing unit (30, 30') is configured to generate a voice command based on the received voice signal (202, 202'),
- wherein the voice processing unit (30, 30') comprises predetermined command information stored internally within the voice processing unit (30,30'),
- wherein the voice processing unit (30, 30') is configured to match the voice command generated from the voice signal (202, 202') with the stored command information to generate a voice command information block,
d. a command processing unit (40, 40') for processing the voice command information block generated by the voice processing unit (30, 30'),
- wherein the command processing unit (40, 40') is connected to the voice processing unit (30, 30') and to the control unit (42, 42'),
- wherein the command processing unit (40, 40') comprises predetermined control information stored within the command processing unit (40, 40') and
- wherein the command processing unit (40, 40') is configured to match the voice command information block with the stored control information to generate a control command and to transmit the control command to the control unit (42, 42').

2. The personal protective device (100, 100', 300, 400, 500) according to claim 1, further comprising a feedback unit (50, 50') comprising at least one indication unit (52, 52', 56, 56', 60, 60') to provide a feedback to the user (200, 200') of the personal protective device (100, 100', 300, 400, 500), wherein the feedback unit (50, 50') is connected to the control unit (42, 42') and wherein the control unit (42, 42') is configured to control the feedback unit (50, 50') such that an indication is provided to the user (200, 200') about the matching of the voice command with the stored command information and/or about the matching of the voice command information block with the stored control information and/or about the control command to be transmitted to the control unit (42, 42').

3. The personal protective device (100, 100', 300, 400, 500) according to claim 2, wherein the feedback unit (50, 50') comprises an audible indication unit (52, 52') to provide an audible indication to a user (200, 200').

4. The personal protective device (100, 100', 300, 400, 500) according to claim 3, wherein the audible indication unit (52, 52') comprises at least one speaker (52, 52') electrically connected to the feedback unit (50, 50') for playing an acoustic signal (204, 204') to the user (200, 200') as audible indication, wherein the control unit (42, 42') is configured to control the feedback unit (50, 50') such that the feedback unit (50, 50') generates an acoustic signal (204, 204') to be played on the at least one speaker (52, 52').

5. The personal protective device (100, 100', 300, 400, 500) according to any one of claims 2 to 4, wherein the feedback unit '(50, 50') comprises a visual indication unit (56, 56') to provide visual indication to a user (200, 200').

6. The personal protective device (100, 100', 300, 400, 500) according to any one of claims 2 to 5, wherein the feedback unit (50. 50') comprises a haptic indication unit (60, 60') to provide haptic indication to a user (200, 200').

7. The personal protective device (100, 300, 400, 500) according to any of the preceding claims, wherein the voice processing unit and/or command processing unit (30, 40) is contained within the personal protective device (100, 300, 400, 500).

8. The personal protective device (100', 300, 400, 500) according to any one of claims 1 to 6, wherein the voice processing unit and/or command processing unit (30', 40') is arranged remote from the personal protective device (100', 300, 400, 500) and wherein the personal protective device (100', 300, 400, 500) is configured to communicate with the voice and/or command processing unit (30', 40') via a short-range connection (22', 32').

9. The personal protective device (100, 100', 300, 400, 500) according to any one of the preceding claims, wherein the voice processing unit (30, 30') comprises storage space for storing additional command information different from the predetermined command information and/or wherein the command processing unit (40, 40') comprises storage space for storing additional control information.

10. The personal protective device (100, 100', 300, 400, 500) according to any one of the preceding claims, wherein the personal protective device (100, 100', 300, 400, 500) is a hearing protection device (400) with local voice recognition further comprising two earmuffs (412, 414) or ear plugs and noise reduction means, optionally a headband (416).

11. The personal protective device (100, 100', 300, 400, 500) according to any one of the preceding claims, wherein the personal protective device (100, 100', 300, 400, 500) is a welding shield (300) with local voice recognition further comprising an automatic darkening filter (330).

12. The personal protective device (100, 100', 300, 400, 500) according to any one of the preceding claims, wherein the personal protective device (100, 100', 300, 400, 500) is a respiratory device (500) with local voice recognition further comprising filter cartridges (522, 524) for filtering inhaled air.

13. The personal protective device (100, 100', 300, 400, 500) according to any one of the preceding claims, wherein the personal protective device (100, 100', 300, 400, 500), preferably the control unit (42, 42'), is further configured to process a keyword training mode for training a user (200, 200') about the correct pronunciation of the voice signals (202, 202').

14. Method of processing a voice signal (202, 202') in a personal protective device (100, 100', 300, 400, 500) with local voice recognition comprising
a. a voice receiving unit (20, 20') comprising at least one microphone (10, 10') for recognizing voice signals (202, 202') of a user (200, 200'),
b. a control unit (42, 42') for controlling functions of the personal protective device (100, 100', 300, 400, 500),
c. a voice processing unit (30, 30') for processing voice signals (202, 202') received from the at least one microphone (10, 10') of the voice receiving unit (20, 20'),
∘ wherein the voice processing unit (30, 30') is connected to the at least one microphone (10, 10'),
∘ wherein the voice processing unit (30, 30') is configured to generate a voice command based on the received voice signal (202, 202'),
∘ wherein the voice processing unit (30, 30') comprises predetermined command information stored internally within the voice processing unit (30, 30'),
∘ wherein the voice processing unit (30, 30') is configured to match the voice command generated from the voice signal (202, 202') with the stored command information to generate a voice command information block,
d. a command processing unit (40, 40') for processing the voice command information block generated by the voice processing unit (30,30'),
∘ wherein the command processing unit (40, 40') is connected to the voice processing unit (30, 30') and to the control unit (42, 42'),
∘ wherein the command processing unit (40, 40') comprises predetermined control information stored within the command processing unit (40, 40') and
∘ wherein the command processing unit (40, 40) is configured to match the voice command information block with the stored control information to generate a control command and to transmit the control command to the control unit (42, 42'),
the method comprising the steps of
I. recognizing a voice signal (202, 202') by a microphone (10, 10'),
II. transmitting the voice signal (202, 202') to the voice processing unit (30, 30'), optionally of an external device (31'),
III. processing the received voice signal (202, 202') with the voice processing unit (30, 30') to generate a voice command based on the received voice signal (202, 202'),
IV. processing the voice command generated from the voice signal (202, 202') and matching it with the command information stored within the voice processing unit (30, 30') to generate a voice command information block,
V. transmitting the voice command information block to the command processing unit (40, 40'),
VI. processing the voice command information block and matching it with the control information stored within the command processing unit (40, 40') to generate a control command and
VII. transmitting the control command to the control unit (42, 42').

15. Method according to claim 14, further comprising the step of providing an indication to the user (200, 200') about the matching of the voice command with the stored command information and/or about the matching of the voice command information block with the stored control information and/or about the control command to be transmitted to the control unit (42, 42'), wherein the indication to the user (200, 200') comprises an audible indication, a visual indication, a haptic feedback or a combination thereof.
